# EUROPÄISCHE PATENTSCHRIFT

(11) **EP 2 237 791 B1**
(45) Veröffentlichungstag und Bekanntmachung des Hinweises auf die Patenterteilung: **20.02.2013**
(21) Anmeldenummer: 08864815.9
(22) Anmeldetag: 22.12.2008
(51) Int. Cl.: A61K 36/899, A23L 1/30, A24B 15/16, A23G 3/48

(54) **GERSTEN- ODER HAFEREXTRAKT ZUR RAUCHERENTWÖHNUNG**
MEANS FOR SMOKING CESSATION
PRODUIT POUR LE SEVRAGE TABAGIQUE

(30) Priorität: 22.12.2007 DE 102007063123
(43) Veröffentlichungstag der Anmeldung: 13.10.2010
(73) Patentinhaber: Ds-pharma Gmbh, 49477 Ibbenbüren (DE)
(72) Erfinder: PODPETSCHNIG - FOPP, Elke, 49545 Tecklenburg (DE)
(74) Vertreter: Simandi, Claus
(86) Internationale Anmeldenummer: PCT/DE2008/002114
(87) Internationale Veröffentlichungsnummer: WO 2009/080004

(56) Entgegenhaltungen:
- DE-A1- 2 311 552
- US-A- 3 862 317
- US-A- 5 234 947
- DATABASE WPI Week 198945 Thomson Scientific, London, GB; AN 1989-328493 XP002229038 & JP 01 243945 A (LOTTE CO LTD) 28. September 1989 (1989-09-28)
- PANDA K ET AL: "Vitamin C prevents cigarette smoke-induced oxidative damage in vivo", FREE RADICAL BIOLOGY AND MEDICINE, ELSEVIER SCIENCE, US, vol. 29, no. 2, 15 July 2000 (2000-07-15), pages 115-124, XP027226486, ISSN: 0891-5849 [retrieved on 2000-07-15]
- KOHEN R ET AL: "PREVENTION OF OXIDATIVE DAMAGE IN THE RAT JEJUNAL MUCOSA BY PECTIN", BRITISH JOURNAL OF NUTRITION, CAMBRIDGE UNIVERSITY PRESS, CAMBRIDGE, GB, vol. 69, no. 3, 1 January 1993 (1993-01-01), pages 789-800, XP009014687, ISSN: 0007-1145, DOI: 10.1079/BJN19930079

## Beschreibung

Die Erfindung betrifft ein Nahrungsergänzungsmittel zur Raucherentwöhnung enthaltend einen Getreideextrakt und dessen Verwendung zur Raucherentwöhnung.

Die Nicotinabhängigkeit ist die Gewöhnung und Abhängigkeit an den Stoff Nicotin im Zuge des Konsums von Tabakprodukten (wie Tabakrauchen, vor allem Zigaretten, Zigarillos oder Zigarren, Tabakspfeifen und Schnupf- und Kautabak sowie beim Rauchen von mit Tabak vermischtem Cannabis). Es können sowohl physische (körperliche) wie auch psychische (geistige / seelische) Symptome auftreten. Die besondere Wirkung des Nicotins auf das Gehirn besteht in einer Catecholamin-Freisetzung in den so genannten Belohnungsarealen der Großhirnrinde und Nicotin bindet an die nicotinergenen Acetylcholinrezeptoren unter Induktion der Ausschüttung verschiedener Neurotransmitter, wie Dopamin, Serotonin, Noradrenalin und Endorphinen. Dies in Verbindung mit dem sensiblen oralen Reiz des Rauchens bewirkt unter Lustgewinn die "positiven" Gefühle des Rauchens.

Die Raucherentwöhnung dient zur Behandlung der Nicotinabhängigkeit oder der Nicotinsucht. Daher treten bei der Raucherentwöhnung charakteristische Entzugserscheinungen in Form von Abstinenzsymptomen auf (Parrot AC, Does Cigarette Smoking Cause Stress? Am. Psyc. 1999 Oct. Vol 54(10) 817-820) und zwar solche wie Unruhezustände, Erregbarkeit, depressive Verstimmung und Müdigkeit.

Im Stand der Technik sind zur Raucherentwöhnung bereits Nicotinpflaster u.ä. Systeme beschrieben.

Ferner ist bekannt, dass spezielle "Raucherdiäten" oder eine gezielte Ernährungsumstellung, die Entzugserscheinungen mildern, da insbesondere der Blutzuckerspiegel stabilisiert werden muss und Neurotransmitter, die durch das Nicotin zuvor verstärkt ausgeschüttet wurden, zu ersetzen sind. Die Raucherentwöhnung ist jedoch zumeist nachteilig von einer Gewichtszunahme begleitet, so dass die Rückfallrate hoch ist.

Zudem ist für Raucher der oxidative Stress im Organismus samt Folgeerkrankungen beschrieben, wie assoziierter Arteriosklerose, Herz-Kreislauf-Erkrankungen (Bergmann S. et al., Systematische Entzündungsmarker und oxidativer Stress bei männlichen Patienten mit koronarchirurgischen Eingriffen: Einfluss des Raucherstatus, Pneumologie 2005, 59). Daher ist der Bedarf an Antioxidantien von Rauchern im Vergleich zu Nichtrauchern massiv erhöht und es werden größere Mengen - bis zu mehr als 200 mg/d oder gar die doppelte Menge - an Carotinoiden und Vitamin C für gleiche Plasmakonzentrationen benötigt (Schectman G, Byrd JC, Hoffmann R, Ascorbic acid Requirements for Smokers: Analysis of a Population Survey, 1991, Am J Nutr 53: 1466-1470). Raucher benötigen die vermehrte Zufuhr von Gemüse, Früchten, anti-oxidativen Vitaminen und sekundären Pflanzenstoffen wie Flavonoiden und Phenolsäuren (Siehe Empfehlung gemäß Biesalski et al, Ernährungsmedizin, Georg Thieme Verlag Stuttgart - New York 1995 Nov (94): 38, 371). Raucher zeigen zudem Mangelerscheinungen an Zink, Calcium, Lycopin und essentiellen Fettsäuren, insbesondere an Omega-3 und Omega-6 Fettsäuren (Schectman (supra), Rao AV, Agarwal S, Effect of Diet and Smoking on Serum Lycopene and Lipid Peroxidation, Nutr. Res. 1998; 18: 713-721).

Einen Einfluss auf das Rauchen ist für den Hafer bereits beschrieben und zwar mittels Einnahme alkoholischer Haferextrakte, die das Verlangen (Craving) nach Zigaretten reduzieren (Anand CL, Effect of Avena sativa on Cigarette Smoking, Nature 1971, 233: 496).

Ein hierzu geeigneter Inhaltsstoff ist das Tryptophan-Analogon und Indolalkaloid Gramin 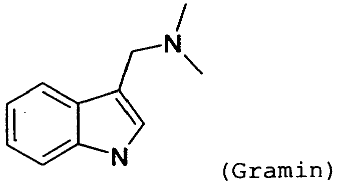 der in Gerste beschrieben worden ist und ein serotoninähnlicher Agonist ist.

Es besteht jedoch ein hohes Bedürfnis und die Aufgabe ein Nahrungsergänzungsmittel bereitzustellen, dass die Raucherentwöhnung zumindest unterstützt.

Die Aufgabe wird durch die Verwendung eines Nahrungsergänzungsmittels nach Anspruch 1 gelöst. Der Getreideextrakt ist ausgewählt aus der Gruppe Gerste und Hafer, bevorzugt Grünhafer. Besonders vorteilhaft wird mittels des bereitgestellten Nahrungsergänzungsmittels eine Gewichtszunahme während der Raucherentwöhnung verhindert oder vermieden (nachstehend erfindungsgemäßes Nahrungsergänzungsmittel).

Die erfindungsgemäßen Nahrungsergänzungsmittel weisen vorteilhafte Mengen an Gramin auf, wobei Gramin mit anderen Inhaltstoffen im Getreideextrakt synergetisch auf die Raucherentwöhnung wirkt. Daher ist erfindungsgemäß ein Grünhaferextrakt besonders bevorzugt.

In einer bevorzugten Ausführungsform ist das erfindungsgemäße Nahrungsergänzungsmittel mit weiteren vorteilhaften Substanzen angereichert, wie Antioxidantien und zwar vorzugsweise Vitamin B und E, sowie Ascorbinsäure (Vitamin C) und / oder Ballaststoffen, insbesondere Pektine, insbesondere Apfelpektine. Insbesondere Vitamin C wirkt dem oxidativen Stress entgegen.

Erfindungsgemäß sollen die Ballaststoffe vorteilhaft unerwünschte Begleitstoffe - wie Schwelstoffe, Schwermetalle - des Tabakkonsums binden können. Besonders erfindungsgemäß geeignet sind Pektine, insbesondere Apfelpektin.

In einer weiteren bevorzugten Ausführungsform ist das erfindungsgemäße Nahrungsergänzungsmittel mit Spurenelementen angereichert, vorzugsweise Chrom (III) Verbindungen, insbesondere Chrom (III) picolinat, Chrom (III) chlorid. Erfindungsgemäß dienen vorzugsweise bis zu 50 µg - 100 µg von Spurenelementen, insbesondere Chrom (III) Verbindungen zur Förderung der Gewichtsabnahme.

In einer weiteren bevorzugten Ausführungsform können weitere pflanzliche Stoffe zur Unterstützung der Raucherentwöhnung in das erfindungsgemäße Nahrungsergänzungsmittel zugefügt werden, wie nicht abschließend mindestens ein pflanzlicher Stoff ausgewählt aus Alfalfa, Baldrian, Eisenkraut, Eukalyptus, Gotu Kola, Helmkraut, Hopfen, Johanniskraut, Süßholz, Passionsblumengewächse und Zitronenmelisse.

In einer bevorzugten Ausführungsform liegt der Getreideextrakt ausgewählt aus der Gruppe Gerste und / oder Hafer, insbesondere Grünhafer, in dem erfindungsgemäßen Nahrungsergänzungsmittel mit einem prozentualen Gewichtsanteil in Höhe von 5 - 70 Gew. %, vorzugsweise 5 - 50 Gew. % oder 5 - 30 Gew. %, insbesondere 10 - 25 Gew. % vor.

Haferextrakt enthält physiologisch wertvolle Nährstoffe dieses Getreides in konzentrierter Form. Mit Haferextrakt werden Antioxidantien, bestimmte Vitamine (insbesondere aus der Gruppe B und E) und Mineralien während der Raucherkompensation kompensiert.

Bevorzugt enthält das erfindungsgemäße Nahrungsergänzungsmittel einen prozentualen Gewichtsanteil an nativen oder synthetischen Vitamin C in Höhe von 1 - 30 Gew. %, insbesondere 3 - 20 Gew. % und Antioxidantien in Höhe von 1 - 30 Gew. %, insbesondere 3 - 20 Gew. %. Ferner ist bevorzugt, dass ein Ballaststoff, insbesondere Dextrin, Pektin, vorzugsweise Apfelpektin mit einem prozentualen Gewichtsanteil in Höhe von 5 - 30 Gew. %, insbesondere 10 - 25 Gew. % eingesetzt wird. Im Übrigen wird im Sinne der Erfindung unter Ballaststoff ein Polysaccharid, einschließlich Cellulose verstanden.

Mengen auf 100 Gew. % sind Bestandteil eines beliebigen Lebensmittels nach anerkannten Verordnungen (EU: EG Nr. 178/2002: Im Sinne dieser Verordnung sind "Lebensmittel" alle Stoffe oder Erzeugnisse, die dazu bestimmt sind oder von denen nach vernünftigem Ermessen erwartet werden kann, dass sie in verarbeitetem, teilweise verarbeitetem oder unverarbeitetem Zustand von Menschen aufgenommen werden, EU: EG Nr. 1924/2006 Health Claims Verordnung u.v.a.), sei es in fester und / oder flüssiger Form.

Im Rahmen dieser Erfindung wird unter einem Nahrungsergänzungsmittel eine dosierte Einheit enthaltend einen Getreideextrakt ausgewählt aus Gerste (Hordeum vulgare) oder Hafer (Avena sativa) verstanden. Eine solche dosierte Einheit ist beispielsweise eine Formulierung im weitesten Sinne gar eine Arzneiform, insbesondere eine Darreichungsform wie Tablette, Pastille, Pressung, Kapsel, Dragees, Ampullen, Pillen, Suppositorien oder dergleichen. Ebenfalls umfasst sind solche Ausführungsformen enthaltend dosierte Einheiten eines erfindungsgemäßen Nahrungsergänzungsmittels in Form eines Blisters. Weiterhin umfasst sind im weitesten Sinne Lösungen solcher dosierten Einheiten wie Emulsionen, Suspensionen bis hin zum Saft.

Die dosierten Einheiten können Trägerstoffe enthalten, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, Sorbit d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die dosierten Einheiten können mit den üblichen, gegebenenfalls Opakisierungsmittel enthaltenden Überzügen und Hüllen versehen sein und derart zusammengesetzt sein, daß sie den oder die erfindungsgemäßen Nahrungsergänzungsmittel nur oder bevorzugt in einem bestimmten Teil des Intestinaltraktes gegebenenfalls verzögert abgeben, wobei als Einbettungsmassen z.B. Polymersubstanzen und Wachse verwendet werden können. Der oder die Wirkstoffe können gegebenenfalls mit einem oder mehreren der oben angegebenen Trägerstoffe ebenfalls in mikroverkapselter Form vorliegen.
Suppositorien können die üblichen wasserlöslichen oder wasserunlöslichen Trägerstoffe enthalten, z.B. Polyethylenglykole, Fette, z.B. Kakaofett und höhere Ester (z.B. C14-Alkohol mit C16-Fettsäure) oder Gemische dieser Stoffe.

In einer ganz besonders bevorzugten Ausführungsform liegt das erfindungsgemäße Nahrungsergänzungsmittel in Form einer Kaumasse oder Lutschkörper vor. Durch die Verwendung einer Kaumasse oder Lutschkörper erfolgt eine Ablenkung von den Abstinenzsymptomen und es wird die Raucherentwöhnung positiv beeinflusst und der Akt des Kauens setzt orale Reize, die den Verlust der rauchergewohnten oralen Reize kompensiert. Ferner erfolgt eine kontinuierliche und schnelle Aufnahme der Inhaltsstoffe, z.B. bereits über die Mundschleimhaut.

Weiterhin können beliebige Aromen zugesetzt werden.

Ferner kann das erfindungsgemäße Nahrungsergänzungsmittel Bestandteil z.B. einer Diät im Rahmen einer Ernährungsmedizin sein. Daher betrifft die Erfindung ebenfalls die Verwendung eines erfindungsgemäßen Nahrungsergänzungsmittels in der Ernährungsmedizin, insbesondere in einer Diät.

Die Dosierungen können 100 mg - 100 g / Tag betragen, insbesondere 200 mg bis 10 g / Tag (durchschnittlicher Erwachsener).

In einer weiteren Ausführungsform der Erfindung betreffen die genannten Ausführungsformen eine Zusammensetzung enthaltend ein erfindungsgemäßes Nahrungsergänzungsmittel zur Behandlung und Prophylaxe von Abstinenzsyndrom, Nicotin-Entzugssymptomen, oxidativen Stress und Nicotinabhängigkeit. Im weitesten Sinne ist das erfindungsgemäße Nahrungsergänzungsmittel ein Functional Food.

Daher betrifft die Erfindung ebenfalls ein Arzneimittel oder pharmazeutische Zusammensetzung bestehend aus einer erfindungsgemäßen Zusammensetzung, insbesondere ein Grünhaferextrakt ebenfalls nach einem der oben genannten Ausführungsformen, zur Behandlung und Prophylaxe von Abstinenzsyndrom, Nicotin-Entzugssymptomen, oxidativen Stress und Nicotinabhängigkeit samt Träger- und Hilfsstoffen. Entsprechende Arzneiformen sind oben genannt.

Nachfolgende Beispiele dienen zur Erläuterung der Erfindung, ohne die Erfindung auf diese Beispiele zu beschränken.

### Beispiel 1:

### Ausführungsform einer erfindungsgemäßen Zusammensetzung

**Tabelle 1:**

| Zutat | mg/Dosis bzw. Einheit |
|---|---|
| Mannit (E 421) | 881,088 |
| **Grünhaferextrakt** | **300,000** |
| Sorbit (E 420) | 300,000 |
| **Apfelpektin** | **200,000** |
| Mikrokristalline Cellulose (E 460) | 162,940 |
| Mono- und Diglyceride von Speisefettsäuren (E 471) | 105, 000 |
| Zitronensäure (E 330) | 75,000 |
| Magnesiumstearat (Trennmittel) (470b) | 33,000 |
| **Ascorbinsäure (Vitamin C) (E 300)** | **15,000** |
| Siliciumdioxid (E 551) | 13,000 |
| Lakritzaroma oder Himbeeraroma oder Aroma | 10, 500 |
| Pfefferminzaroma oder Aroma | 4,200 |
| Ethylcellulose (E 462) | 0,375 |

### Beispiel 2:

### Studienergebnisse zeigen die vorteilhafte Eignung von Grünhafer zur Raucherentwöhnung (8 bis 15 Probanden).

Figur la zeigt eine signifikante Reduktion des Raucherkonsums bei regelmäßiger Einnahme eines Grünhaferextraktes. Insbesondere tritt eine weitere Verbesserung der Werte bei einer Dosierung nach Beispiel 1 ein. Die Dosierung des Grünhaferextraktes kann z.B. zwischen 100 - 500 mg/Tag variieren. Vorzugsweise 3x täglich.
Legende: Y- Achse Zigaretten / Tag in 5-er Einheiten, 5 - 25; X- Achse Zeit in Tagen / 1 bis 28 Tagen

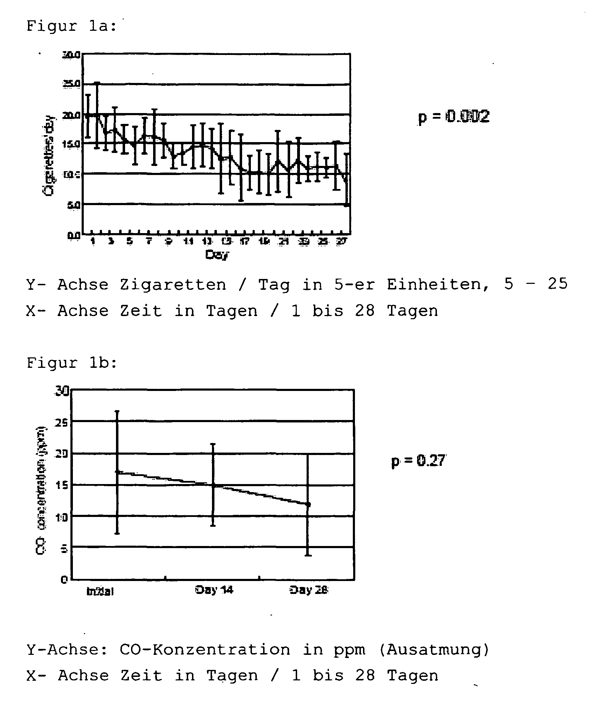

Figur 1b zeigt die signifikante Reduktion von Kohlenmonoxid im Atmen der Probanden.
Legende: Y-Achse: CO-Konzentration in ppm (Ausatmung)
X- Achse Zeit in Tagen / 1 bis 28 Tagen

## Patentansprüche

1. Nahrungsergänzungsmittel enthaltend einen Getreideextrakt aus Gerste zur Anwendung bei der Raucherentwöhnung.

2. Nahrungsergänzungsmittel nach Anspruch 1, enthaltend zusätzlich Antioxidantien, Vitamin C und / oder Ballaststoffe, insbesondere Pektin.

3. Nahrungsergänzungsmittel enthaltend einen Getreideextrakt aus Hafer, insbesondere Grünhafer, und zusätzlich Antioxidantien, Vitamin C und / oder Ballaststoffe, insbesondere Pektin zur Anwendung bei der Raucherentwöhnung.

4. Nahrungsergänzungsmittel nach Anspruch 1 bis 3, enthaltend zusätzlich mindestens einen pflanzlichen Stoff ausgewählt aus Alfalfa, Baldrian, Eisenkraut, Eukalyptus, Gotu Kola, Helmkraut, Hopfen, Johanniskraut, Süßholz, Passionsblumengewächse und Zitronenmelisse.

5. Nahrungsergänzungsmittel nach einem der vorhergehenden Ansprüche, enthaltend zusätzlich Spurenelemente, insbesondere Chrom (III) Verbindungen.

6. Nahrungsergänzungsmittel nach einem der vorhergehenden Ansprüche, enthaltend einen Getreideextrakt mit einem prozentualen Gewichtsanteil in Höhe von 5 - 70 Gew. %, 5 - 50 Gew. %, 5 - 30 Gew. %, insbesondere 10 - 25 Gew. %.

7. Nahrungsergänzungsmittel nach einem der vorhergehenden Ansprüche,
**dadurch gekennzeichnet, dass** es in Form einer dosierten Einheit vorliegt, wie Tablette, Pastille, Pressung, Kapsel, Dragees, Ampullen, Pillen, Suppositorien, ggfs. weitere Träger- und Hilfsstoffe, ggfs. in Form eines Blisters
oder
in Form einer Lösung, Emulsion, Suspension, Saft.

8. Nahrungsergänzungsmittel nach einem der vorhergehenden Ansprüche, **dadurch gekennzeichnet, dass** es in Form einer Kaumasse und / oder Lutschkörper vorliegt.

9. Nahrungsergänzungsmittel nach einem der vorhergehenden Ansprüche zur Anwendung in der Ernährungsmedizin, insbesondere in einer Diät.

10. Zusammensetzung enthaltend ein Nahrungsergänzungsmittel nach einem der vorhergehenden Ansprüche zur Anwendung bei Abstinenzsyndrom, Nicotin-Entzugssymptomen, oxidativen Stress und Nicotinabhängigkeit.

11. Arzneimittel oder pharmazeutische Zusammensetzung gemäß einer Zusammensetzung nach Anspruch 11 zur Anwendung bei Abstinenzsyndrom, Nicotin-Entzugssymptomen, oxidativem Stress und Nicotinabhängigkeit samt Träger- und Hilfsstoffen.

## Claims

1. A dietary supplement containing a grain extract of barley for use in smoking cessation.

2. The dietary supplement according to claim 1, containing additional antioxidants, vitamin C and/or bulk, in particular pectin.

3. The dietary supplement containing a grain extract of oat, in particular green oat, and additional antioxidants, vitamin C and/or bulk, in particular pectin for use in smoking cessation.

4. The dietary supplement according to claims 1 to 3, also containing at least one vegetable substance selected from alfalfa, valerian, verbena, eucalyptus, gotu kola, scutellaria, hop, St. John's wort, liquorice, passion flower plants and lemon balm.

5. The dietary supplement according to one of the preceding claims, also containing trace elements, in particular chromium (III) compounds.

6. The dietary supplement according to one of the preceding claims, containing a grain extract having a percentage by weight of 5-70% by weight, 5-50% by weight, 5-30% by weight, in particular 10-25% by weight.

7. The dietary supplement according to one of the preceding claims, **characterized in that** this is present in the form of a metered unit, such as tablets, pastilles, pressing, capsules, dragées, ampules, pills, suppositories, optionally further carrier or auxiliary substances, optionally in the form of a blister or in the form of a solution, emulsion, suspension, juice.

8. The dietary supplement according to one of the preceding claims, **characterized in that** this is present in the form of a chewable mass and/or lozenge.

9. The dietary supplement according to one of the preceding claims for use in nutritional medicine, in particular in a diet.

10. A composition containing a dietary supplement according to one of the preceding claims for use in abstinence syndrome, nicotin-withdrawal symptoms, oxidative stress and nicotine dependence.

11. A pharmaceutical or a pharmaceutical composition according to a composition according to claim 11 for use in abstinence syndrome, nicotin-withdrawal symptoms, oxidative stress and nicotine dependence, which includes carrier and auxiliary substances.

## Revendications

1. Complément alimentaire contenant un extrait de grain à base d'orge, destiné à une utilisation dans le cadre d'un sevrage tabagique.

2. Complément alimentaire selon la revendication 1, contenant en outre des antioxydants, de la vitamine C et/ou des fibres alimentaires, en particulier de la pectine.

3. Complément alimentaire contenant un extrait de grain à base d'avoine, en particulier d'avoine verte, ainsi que des antioxydants, de la vitamine C et/ou des fibres alimentaires, en particulier de la pectine, destiné à une utilisation dans le cadre d'un sevrage tabagique.

4. Complément alimentaire selon l'une des revendications 1 à 3, contenant en outre au moins une substance végétale sélectionnée parmi l'alfalfa, la valériane, la verveine officinale, l'eucalyptus, le gotu kola, la scutellaire, le houblon, le mille-pertuis, le bois de réglisse, la passifloracée et la mélisse officinale.

5. Complément alimentaire selon l'une des revendications précédentes, contenant en outre des oligoéléments, en particulier les composés du chrome (III).

6. Complément alimentaire selon l'une des revendications précédentes, contenant un extrait de grain avec un pourcentage en poids à hauteur de 5-70% en poids, de 5-50% en poids, de 5-30% en poids, en particulier de 10-25% en poids.

7. Complément alimentaire selon l'une des revendications précédentes, **caractérisé en ce qu'**il se présente sous la forme d'une dose individuelle, telle qu'une tablette, une pastille, un comprimé, une capsule, une dragée, une ampoule, une pilule, un suppositoire, éventuellement d'autres excipients, éventuellement sous la forme d'un blister ou sous la forme d'une solution, d'une émulsion, d'une suspension, d'un jus.

8. Complément alimentaire selon l'une des revendications précédentes, **caractérisé en ce qu'**il se présente sous la forme d'une masse à mastiquer et/ou d'un corps à sucer.

9. Complément alimentaire selon l'une des revendications précédentes, destiné à une utilisation dans le cadre de la médecine nutritionnelle, en particulier d'un régime.

10. Composition contenant un complément alimentaire selon l'une des revendications précédentes, destinée à une utilisation dans le cadre du syndrome d'abstinence, de symptômes de sevrage nicotinique, du stress oxydant et de la dépendance à la nicotine.

11. Médicament ou composition pharmaceutique selon une composition selon la revendication 11, destiné à une utilisation dans le cadre du syndrome d'abstinence, de symptômes de sevrage nicotinique, du stress oxydant et de la dépendance à la nicotine, y compris les excipients.
